(19) Europäisches Patentamt
European Patent Office
Office européen des brevets

(11) Veröffentlichungsnummer: **0 025 106**
**B1**

(12) # EUROPÄISCHE PATENTSCHRIFT

(45) Veröffentlichungstag der Patentschrift: **22.09.82**

(51) Int. Cl.³: **C 07 C143/38**

(21) Anmeldenummer: **80104420.7**

(22) Anmeldetag: **26.07.80**

(54) **Verfahren zur Herstellung von gegebenenfalls zumindest teilweise als Salz einer anorganischen oder organischen Base vorliegenden Isocyanatoarylsulfonsäuren.**

(30) Priorität: **08.08.79 DE 2932094**

(43) Veröffentlichungstag der Anmeldung: **18.03.81 (Patentblatt 81/11)**

(45) Bekanntmachung des Hinweises auf die Patenterteilung: **22.09.82 Patentblatt 82/38**

(84) Benannte Vertragsstaaten: **BE DE FR GB IT NL**

(56) Entgegenhaltungen:
**DE - A - 2 227 111**
**DE - A1 - 2 615 876**

(73) Patentinhaber: **BAYER AG**
**Zentralbereich Patente, Marken und Lizenzen**
**D-5090 Leverkusen 1, Bayerwerk (DE)**

(72) Erfinder: **Dieterich, Dieter, Dr.**
**Ludwig-Girtler-Strasse 1**
**D-5090 Leverkusen (DE)**

**EP 0 025 106 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren zur Herstellung von gegebenenfalls zumindest teilweise als Salz einer anorganischen oder organischen Base vorliegende Isocyanatoarylsulfonsäuren

Die vorliegende Erfindung betrifft ein neues Verfahren zur Herstellung von gegebenenfalls zumindest teilweise als Salz einer anorganischen oder organischen Base vorliegenden Isocyanatoarylsulfonsäuren durch Sulfonierung der entsprechenden Carbamidsäurechloride, Überführung der Carbamidsäurechloridgruppen in Isocyanatgruppen und gegebenenfalls anschließende Neutralisation zumindest eines Teils der vorliegenden Sulfonsäuregruppen.

Isocyanatoarylsulfonsäuren, d.h. aromatische Isocyanate, welche Sulfonsäuregruppen enthalten, sind bekannt. Bei diesen bekannten Verbindungen handelt es sich entweder um definierte Mono- oder Polysulfonsäuren (vgl. DE-OS 19 39 911, DE-OS 25 24 476, DE-OS 26 15 876) oder um Polyisocyanatgemische, welche oft nur anteilig Isocyanatoarylsulfonsäure enthalten (DE-OS 22 27 111, DE-OS 23 59 614, DE-OS 23 59 615). Bislang wurden diese Produkte durch Sulfonierung der entsprechenden Isocyanate, vorzugsweise mit Schwefeltrioxid, hergestellt. Die Isocyanatgruppen liegen in diesen Sulfonierungsprodukten oft in Form ihrer Dimeren (Uretdione) vor.

Überraschenderweise wurde nunmehr gefunden, daß zur Herstellung von Isocyanatoarylsulfonsäuren eine Isolierung bzw. Reindarstellung der entsprechenden Arylisocyanate nicht erforderlich ist, sondern daß man die Isocyanatoarylsulfonsäuren in besonders guten Ausbeuten auch durch Sulfonierung der entsprechenden Carbamidsäurechloride unter gleichzeitiger oder anschließender Chlorwasserstoffabspaltung herstellen kann. Dies bedeutet eine wesentliche Vereinfachung der bisherigen Verfahren zur Herstellung von Isocyanatoarylsulfonsäuren durch Sulfonierung der entsprechenden Isocyanate, da beim Arbeiten gemäß dem erfindungsgemäßen Prinzip die Herstellung der Arylisocyanate durch Phosgenierung der entsprechenden Arylamine mit der Sulfonierungsreaktion kombiniert werden kann. Besonders überraschend war in diesem Zusammenhang die Beobachtung, daß die Chlorwasserstoffabspaltung aus den sulfonierten Carbamidsäurechloriden glatter und einfacher verläuft als die Abspaltung von Chlorwasserstoff aus den entsprechenden unsulfonierten Carbamidsäurechloriden, wie sie bei der Phosgenierung der entsprechenden Amine als Zwischenprodukt anfallen. Die nach dem geschilderten Prinzip erhältlichen Isocyanatoarylsulfonsäuren können durch einfache Neutralisation zumindest eines Teils der vorliegenden Sulfonsäuregruppen in die entsprechenden zumindest teilweise in der Salzform vorliegenden Derivate überführt werden.

Gegenstand der vorliegenden Erfindung ist somit ein Verfahren zur Herstellung von gegebenenfalls zumindest teilweise als Salz einer anorganischen oder organischen Base vorliegenden Isocyanatoarylsulfonsäuren, dadurch gekennzeichnet, daß man aromatische Carbamidsäurechloride bei − 10 °C bis 140 °C mit Schwefeltrioxid oder Chlorsulfonsäure zur Reaktion bringt, gleichzeitig oder anschließend die vorliegenden Carbamidsäurechlorid-Gruppen durch eine Hitzebehandlung bei 40 °C bis 180 °C unter Abspaltung von Chlorwasserstoff in Isocyanatgruppen überführt und schließlich gegebenenfalls zumindest einen Teil der vorliegendenden Sulfonsäuregruppen durch Neutralisation mit einer anorganischen oder organischen Base in Sulfonatgruppen überführt.

In den erfindungsgemäßen Verfahrensprodukten liegen die Isocyanatgruppen oft zumindest teilweise in dimerisierter Form, d.h. als Uretdiongruppen vor. Insbesondere bei der Sulfonierung von Aryl-monocarbamidsäurechloriden können die durch die Sulfonierungsreaktion eingeführten Sulfonsäuregruppen auch zumindest teilweise in Form der entsprechenden Anhydridgruppen vorliegen. Der Ausdruck « Isocyanatoarylsulfonsäuren » umfaßt somit im Rahmen der vorliegenden.

Erfindung wohl die entsprechenden freie Sulfonsäure und freie Isocyanatgruppen aufweisenden Verbindungen als auch die entsprechenden Uretdiongruppen aufweisenden Verbindungen als auch die entsprechenden Sulfonsäureanhydridgruppen aufweisenden Verbindungen. Auch in den zumindest teilweise als Salz vorliegenden Verfahrensprodukten können die Isocyanatgruppen in zumindest teilweise dimerisierter Form als Uretdiongruppen vorliegen.

Ausgangsmaterialien für das erfindungsgemäße Verfahren sind beliebige Arylcarbamidsäurechloride, d.h. beliebige mindestens eine aromatisch gebundene Carbamidsäurechloridgruppe und gegebenenfalls weitere bezüglich der Sulfonierungsreaktion indifferente Substituenten aufweisende aromatische Verbindungen. Vorzugsweise handelt es sich bei den Ausgangsmaterialien für das erfindungsgemäße Verfahren um Verbindungen, welche einen bis vier aromatische Ringe und eine bis vier aromatisch gebundene Carbaminsäurechloridgruppen enthalten und im übrigen den obigen Bedingungen entsprechen. Zu den bevorzugten Ausgangsmaterialien für das erfindungsgemäße Verfahren gehören Carbamidsäurechloride der Formel

$$R(NH—CO—Cl)_n$$

in welcher

R für einen gegebenenfalls Methylsubstituenten und/oder Methylenbrücken aufweisenden n-wertigen aromatischen Kohlenwasserstoffrest mit insgesamt 6 bis 30, vorzugsweise 7 bis 15

Kohlenstoffatomen steht und

n für eine ganze Zahl von 2 bis 4, vorzugsweise 2 steht,

oder beliebige Gemische derartiger Carbamidsäurechloride. In den bevorzugten Carbamidsäurechloriden der genannten allgemeinen Formel weist jeder aromatische Ring mindestens eine und höchstens zwei Carbamidsäurechloridgruppen auf.

Beispiele geeigneter Monocarbamidsäurechloride sind Phenyl-, p-Toluyl-, p-Chlorphenyl-, p-Nitrophenyl-, p-Methoxyphenyl-, m-Chlorphenyl-, m-Chlormethylphenyl- oder p-Chlormethylphenylcarbamidsäurechlorid. Beispiele geeigneter Bis-Carbamidsäurechloride sind Benzol-1,4-bis-carbamidsäurechlorid, Toluol-2,4-bis-carbamidsäurechlorid, Naphthalin-1,5-bis-carbamidsäurechlorid, Diphenylmethan-4,4'- oder 2,4'-bis-carbamidsäurechlorid, Stilben-4,4'-bis-carbamidsäurechlorid, 3,3'-Dichlordiphenylmethan-4,4'-bis-carbamidsäurechlorid, Diphenylpropan-4,4'-bis-carbamidsäurechlorid, Diphenylether-4,4'-bis-carbamidsäurechlorid, Diphenylsulfid-4,4'-bis-carbamidsäurechlorid oder Diphenyl-sulfon-4,4'-bis-carbamidsäurechlorid. Beispiele höherfunktioneller ($n = 3$ oder 4) für das erfindungsgemäße Verfahren geeigneter Carbamidsäurechloride sind insbesondere die bei der Umsetzung von drei- oder vierkernigen Anilin/Formaldehyd-Kondensaten, d.h. den höheren Homologen der Diaminodiphenylmethan-Isomeren mit Phosgen entstehenden Carbamidsäurechloride. Beispiele besonders bevorzugter für das erfindungsgemäße Verfahren geeigneter Carbamidsäurechloride sind Toluol-2,4-bis-carbamidsäurechlorid, Toluol-2,6-bis-carbamidsäurechlorid, bzw. aus diesen Isomeren bestehende Gemische, Diphenylmethan-4,4'-bis-carbamidsäurechlorid, Diphenylmethan-2,4'-bis-carbamidsäurechlorid, aus diesen Isomeren bestehende Gemische, sowie Gemische dieser zuletzt genannten Isomeren mit den beispielhaft genannten höher als difunktionellen Carbamidsäurechloriden, wie sie z.B. bei der Umsetzung von Anilin/Formaldehyd-Kondensaten mit Phosgen erhalten werden.

Bei der Durchführung des erfindungsgemäßen Verfahrens wird als Sulfonierungsmittel Chlorsulfonsäure oder Schwefeltrioxid eingesetzt, wobei das Schwefeltrioxid als Flüssigkeit, in Form einer Lösung oder gasförmig, gegebenenfalls in durch Inertgas wie z.B. Stickstoffverdünnter Form oder in Form einer Additionsverbindung an geeignete organische Verbindungen zum Einsatz gelangen kann. Ein geeignetes Lösungsmittel für das Schwefeltrioxid ist beispielsweise konzentrierte Schwefelsäure, so daß als Sulfonierungsmittel Oleum zum Einsatz gelangt. Additionsverbindungen des Schwefeltrioxids mit geeigneten organischen Verbindungen ist beispielsweise Pyridin-$SO_3$, Dioxan-$SO_3$, Tetrahydrofuran-$SO_3$, Ether-$SO_3$ oder Dimethylformamid-$SO_3$. Bevorzugt wird die Sulfonierung mit gasförmigem, in einem Stickstoffstrom verdünntem Schwefeltrioxid mit Oleum oder mit Chlorsulfonsäure durchgeführt.

Die Sulfonierungsreaktion wird vorzugsweise in Gegenwart von sowohl gegenüber $SO_3$ als auch gegenüber NCO-Gruppen unter den Reaktionsbedingungen chemisch inerten Lösungsmitteln durchgeführt, wie z.B. in Dichlorethan, Tetrachlorethan, Methylenchlorid, Chloroform, Nitromethan, Tetrahydrofuran, Phosphoroxichlorid, Dioxan, Nitrobenzol. In Sonderfällen, insbesondere bei Teilsulfonierung können auch Chlorbenzol und o-Dichlorbenzol eingesetzt werden.

Die Menge an eingesetztem Sulfonierungsmittel beträgt 0,01 bis 6 Mol pro Mol aromatisches Carbamidsäurechlorid. Wird lediglich eine Teilsulfonierung durchgeführt, so beträgt die Menge des Sulfonierungsmittels pro Mol eingesetztes Carbamidsäurechlorid 0,01 bis 0,95 Mol, vorzugsweise 0,05 bis 0,3 Mol. Für die ganz besonders bevorzugte Monosulfonierung werden 0,95 bis 1,3 Mol, vorzugsweise 1,0 bis 1,1 Mol Sulfonierungsmittel eingesetzt. Für eine Polysulfonierung werden 1,3 bis 6,0 Mol, vorzugsweise 2,0 bis 4,0 Mol Sulfonierungsmittel benötigt. Dabei kommen Mengen oberhalb 2,0 Mol praktisch nur dann in Betracht, wenn es sich um Carbamidsäurechloride mit 2 oder 3 aromatischen Ringen handelt.

Die Sulfonierung wird im allgemeinen bei Temperaturen zwischen −10 °C und 140 °C durchgeführt, vorzugsweise zwischen 0 °C und 80 °C. Man kann entweder der Lösung bzw. Suspension des Carbamidsäurechlorids das Sulfonierungsmittel allmählich zugeben oder auch das Carbamidsäurechlorid in vorgelegtes Sulfonierungsmittel einlaufen lassen. Die Sulfonierungsreaktion findet in Lösung praktisch momentan, in Suspension in der Regel innerhalb weniger Minuten statt. Bei der im Rahmen der vorliegenden Erfindung besonders bevorzugten Kombination der Herstellung der Polyisocyanate durch Phosgenierung der entsprechenden Amine mit der Sulfonierungsreaktion erfolgt die erfindungsgemäße Sulfonierung durch Zugabe des Sulfonierungsmittel bei einer Temperatur innerhalb der genannten Bereiche zu dem bei der Phosgenierung der entsprechenden Amine entstehenden Carbamidsäurechlorid und zwar frühestens unmittelbar nach dem Vermischen des Amins mit dem Phosgen und spätestens unmittelbar vor der Überführung der intermediär gebildeten Carbamidsäurechloride in die entsprechenden Isocyanate durch thermische Abspaltung von Chlorwasserstoff.

Im Anschluß an die Sulfonierungsreaktion wird in der für die Isocyanatherstellung üblichen Weise di Dehydrohalogenierung des sulfonierten Carbamidsäurechlorids zum sulfonierten Isocyanat durchgeführt. Hierzu wird das Reaktionsgemisch auf 40 °C bis 180 °C erwärmt, wobei zur Beschleunigung ein inerter Gasstrom (Stickstoff, Kohlendioxid) durch das Reaktionsgemisch geleitet werden kann.

Die Dehydrohalogenierung wird hierbei durch die Sulfonsäuregruppen erleichtert und erfolgt bereits bei tieferer Temperatur als bei unsubstituierten Carbamidsäurechloriden. Bei Polysulfonierung setzt die Abspaltung von Chlorwasserstoff selbst bei Raumtemperatur schon während der Sulfonierung ein.

Die Reaktionsprodukte einer Mono- oder Polysulfonierung sind feste, in den meisten Lösungsmitteln unlösliche Produkte, die von Lösungsmitteln vorzugsweise durch Dekantieren, Filtrieren oder Abschleudern abgetrennt werden können. Es handelt sich dabei in den meisten Fällen um Uretdione die zweckmäßigerweise wegen ihrer extremen Feuchtigkeitsempfindlichkeit nicht getrocknet, sondern noch feucht weiter verarbeitet werden. Wurde nur die Teilsulfonierung durchgeführt, so bleibt die Isocyanatoarylsulfonsäure entweder gelöst oder geht beim Abdestillieren des Lösungsmittels in Lösung. Man erhält dann flüssige modifizierte Isocyanate, wie sie in DE-OS 22 27 111 und DE-OS 23 59 614 beschrieben sind.

Wie bereits ausgeführt, liegen in den erfindungsgemäßen Verfahrensprodukten die Isocyanatgruppen oftmals zumindest teilweise in dimerisierter Form und die Sulfonsäure-gruppen — insbesondere bei der Verwendung von monofunktionellen Carbamidsäurechloriden als Ausgangsmaterial — oftmals zumindest teilweise in Form von Sulfonsäureanhydrid-gruppen vor. Die in den Verfahrensprodukten vorliegenden freien Sulfonsäuregruppen können durch Reaktion mit Basen, insbesondere mit tertiären Aminen ganz oder teilweise in die entsprechenden Sulfonatgruppen überführt werden. Geeignete tertiäre Amine sind beispielsweise Trimethylamin, Triethylamin, Tributylamin, Dimethylanilin oder Pyridin. Die Überführung der Säuren in die entsprechenden Salze geschieht beispielsweise durch Aufschlämmen der Säuren in einem nicht-Lösungsmittel wie z.B. Tetrachlorethan oder Lösen der Säuren in einem Lösungsmittel wie z.B. Aceton und Zugabe des zur Salzherstellung verwendeten tertiären Amins. Die Herstellung der Salze kann aber auch beispielsweise durch Begasen der pulverförmigen Säuren mit gasförmigen tertiären Aminen vorgenommen werden. Die Herstellung von Salzen der erfindungsgemäßen Verfahrensprodukte mit anorganischen Basen geschieht beispielsweise durch Umsetzung der Lösungen der Säuren in beispielsweise Aceton mit Dispersionen von Alkalimetallen, oder Alkalimetallhydriden, insbesondere Dispersionen von Natrium, Natriumhydrid oder Kalium in beispielsweise Toluol.

Die bevorzugten erfindungsgemäßen Verfahrensprodukte sind durch einen Gehalt an, gegebenenfalls in dimerisierter Form vorliegenden, Isocyanatgruppen von 10 bis 42 Gew.-% und einem Gehalt an, in Form vom Sulfonsäure-, Sulfonat- und/oder Sulfonsäureanhydridgruppen vorliegendem Schwefel von 0,5 bis 20 Gew.-% gekennzeichnet.

Die erfindungsgemäßen Verfahrensprodukte eignen sich für alle bislang bekanntgewordenen Einsatzgebiete für Isocyanatoarylsulfonsäuren.

In den nachfolgenden Beispielen beziehen sich alle Prozentangabn auf Gew.-%.

Beispiel 1

123,5 g (0,5 Mol) Toluol-bis-carbamidsäurechlorid (80 % 2,4-, 20 % 2,6-Isomeres) werden in 500 ml 1,2-Dichlorethan suspendiert. In die Suspension werden 42 g (0,525 Mol) Schwefeltrioxid bei 2-10 °C eingeleitet. Anschließend wird langsam unter Rühren erwärmt, wobei ab 50 °C HCl-Abspaltung einsetzt. Bei 85 °C ist die HCl-Abspaltung im wesentlichen beendet. Es wird solange Stickstoff durch die Suspension geblasen, bis das Abgas frei von HCl ist. Dann wird abfiltriert, mit Dichlorethan gewaschen und getrocknet. Es werden 92 g eines Pulvers erhalten, dessen IR-Spektrum dem des nach DE-OS 26 40 103, Beispiel 1 erhaltenen Produkt entspricht. Es handelt sich demnach um das Uretdion der Toluylendiisocyanat-5-sulfonsäure.

Beispiel 2

Dasselbe Produkt wie gemäß Beispiel 1 wird erhalten, wenn man 0,5 Mol eines Isomerengemisches aus 2,4-Diaminotoluol und 2,6-Diaminotoluol (Gewichtsverhältnis = 80 : 20) und 2 Mol Phosgen in 500 ml 1,2-Dichlorethan bei 0 °C innig vermischt, wobei das entsprechende Biscarbamidsäurechlorid entsteht, unter Durchleiten von Phosgen und Erwärmen bei 50-70 °C mit Schwefeltrioxid oder Chlorsulfonsäure sulfoniert und anschließend auf 85 °C erhitzt und mittels Stickstoff Chlorwasserstoff ausbläst.

Beispiel 3

19,8 g (0,1 Mol) 4,4'-Diaminodiphenylmethan in 50 ml 1,2-Dichlorethan werden unter intensivem Rühren in eine auf − 10 °C gekühlte Lösung von 40 g (0,4 Mol) Phosgen in 100 ml 1,2-Dichlorethan eingerührt, wobei das Diphenylmethan-biscarbamidsäurechlorid entsteht. Anschließend werden 12 g (0,1 Mol) Chlorsulfonsäure zugetropft. Unter Durchleiten von Stickstoff wird die Suspension bis zum Sieden erhitzt, wobei der größte Teil des Chlorwasserstoffs entweicht. Der verbleibende Rest wird nach Zusatz von 50 ml 1,2-Dichlorbenzol und Abdestillieren des 1,2-Dichlorethans durch kurzes Erhitzen auf 140 °C entfernt.

Das Reaktionsprodukt entspricht dem gemäß DE-OS 25 24 476, Beispiel 1 erhaltenen. Es handelt sich demnach um das Uretdion der 4,4'-Diisocyanato-diphenylmethan-3-sulfonsäure.

Beispiel 4

100 g eines technischen Polyamingemischs der Diphenylmethanreihe, welches durch eine an sich bekannte säurekatalysierte Anilin/Formaldehyd-Kondensation hergestellt worden ist und welches 44 % Diaminodiphenylmethan-Isomere, 24 % dreikernige Triamine und 32 % höherkernige Homologe enthält, werden in 200 ml Chlorbenzol gelöst.

Diese Lösung wird anschließend unter intensivem Rühren in eine auf 5 °C gekühlte Lösung von 200 g Phosgen in 500 ml Chlorbenzol eingerührt. wobei das entsprechende Carbamidsäurechlorid-Gemisch entsteht. Anschließend werden 4 g Schwefeltrioxid eingeleitet. Das Reaktionsgemisch wird unter Durchleiten von weiterem Phosgen zum Sieden erhitzt und dann solange Stickstoff durchgeleitet, bis kein Chlorwasserstoff mehr entweicht. Nach Abdestillieren des Chlorbenzols wird ein homogenes flüssiges ansulfoniertes Polyisocyanat erhalten.

Viskosität : 830 cP, S-Gehalt 1,3 %

NCO-Gehalt : 31,2 %

**Anspruch**

Verfahren zur Herstellung von gegebenenfalls zumindest teilweise als Salz einer anorganischen oder organischen Base vorliegenden Isocyanatoarylsulfonsäuren, dadurch gekennzeichnet, daß man aromatische Carbamidsäurechloride bei − 10 °C bis 140 °C mit Schwefeltrioxid oder Chlorsulfonsäure zur Reaktion bringt, gleichzeitig oder anschließend die vorliegenden Carbamidsäurechlorid-Gruppen durch eine Hitzebehandlung bei 40 °C bis 180 °C unter Abspaltung von Chlorwasserstoff in Isocyanatgruppen überführt und schließlich gegebenenfalls zumindest einen Teil der vorliegendenden Sulfonsäuregruppen durch Neutralisation mit einer anorganischen oder organischen Base in Sulfonatgruppen überführt.

**Claim**

Process for the production of isocyanatoaryl sulphonic acids optionally present at least partly as salts of an inorganic or organic base, characterised in that aromatic carbamic acid chlorides are reacted with sulphur trioxide or chlorosulphonic acid at a temperature of from − 10 °C to 140 °C, the carbamic acid chloride groups present are simultaneously or subsequently converted into isocyanate groups by a heat treatment at a temperature of from 40 °C to 180 °C with the evolution of hydrogen chloride and finally, optionally, at least some of the sulphonic acid groups present are converted into sulphonate groups by neutralisation with an inorganic or organic base.

**Revendication**

Procédé pour la préparation d'acides isocyanatoarylsulfoniques éventuellement présents au moins partiellement sous forme de sel d'une base organique ou inorganique, caractérisé en ce que l'on fait réagir des chlorures d'acides carbamiques aromatiques avec le trioxyde de soufre ou l'acide chlorosulfonique à − 10 à 140 °C, on transforme simultanément ou ensuite les groupes chlorures d'acides carbamiques présents en groupes isocyanates par un traitement thermique à 40-180 °C avec élimination de chlorure d'hydrogène, et enfin on transforme éventuellement au moins une partie des groupes acides sulfoniques présents en groupes sulfonates par neutralisation avec une base inorganique ou organique.